# EUROPÄISCHE PATENTSCHRIFT

(11) **EP 2 934 381 B1**
(45) Veröffentlichungstag und Bekanntmachung des Hinweises auf die Patenterteilung: **05.09.2018**
(21) Anmeldenummer: 13805865.6
(22) Anmeldetag: 13.12.2013
(51) Int. Cl.: A61F 2/16

(54) **INTRAOKULARLINSEN-AUFBEWAHRUNGSSYSTEM**
INTRAOCULAR LENS STORAGE SYSTEM
SYSTÈME DE CONSERVATION DE LENTILLES INTRAOCULAIRES

(30) Priorität: 20.12.2012 DE 102012223885
(43) Veröffentlichungstag der Anmeldung: 28.10.2015
(73) Patentinhaber: HumanOptics AG, 91054 Erlangen (DE)
(72) Erfinder: MESSNER, Arthur, 91220 Schnaittach (DE); HEISS, Martin Christoph, 9050 Eggerstanden (CH)
(74) Vertreter: Rau, Schneck & Hübner Patentanwälte Rechtsanwälte PartGmbB
(86) Internationale Anmeldenummer: PCT/EP2013/076504
(87) Internationale Veröffentlichungsnummer: WO 2014/095611

(56) Entgegenhaltungen:
- EP-A1- 2 286 762
- EP-A1- 2 286 764
- WO-A1-2006/070219
- DE-A1-102006 000 929
- FR-A1- 2 875 125

## Beschreibung

Die Erfindung betrifft ein Intraokularlinsen-Aufbewahrungssystem zur Aufbewahrung einer Intraokularlinse gemäß dem Oberbegriff des Anspruchs 1. Ferner richtet sich die Erfindung auch auf eine Intraokularlinsen-Übergabe-Anordnung zur Übergabe einer Intraokularlinse an eine Injektions-Einrichtung mit einem entsprechenden Intraokularlinsen-Aufbewahrungssystem. Die Erfindung betrifft außerdem ein Verfahren zur Übergabe einer Intraokularlinse an eine Injektions-Einrichtung unter Bereitstellung eines entsprechenden Intraokularlinsen-Aufbewahrungssystems.

Intraokularlinsen, die künstliche Augenlinsen sind, werden bis zu ihrer Verwendung bzw. Implantation im sterilen Zustand gelagert. Es ist bekannt, dafür sogenannte Primär-Verpackungen heranzuziehen, die sich wiederum in einer Sekundär-Verpackung, wie einer Steril-Tüte, befinden. In den Primär-Verpackungen können fixierte oder lose Intraokularlinsen-Aufnahmen zur Aufnahme der Intraokularlinsen angeordnet sein. Weiterhin ist es bekannt, die Primär-Verpackung selbst oder die Intraokularlinsen-Aufnahme als Teil einer Injektions-Einrichtung oder als komplette Injektions-Einrichtung auszuführen, so dass die Intraokularlinse ohne Manipulation, z.B. mit einer Pinzette, direkt durch die Injektions-Einrichtung implantiert werden kann.

Nachteilig an diesen bekannten Intraokularlinsen-Aufbewahrungssystemen ist, dass häufig Biokompatibilitäts-Probleme auftreten. Diese sind vor allem auf den Einsatz von Gleit-Additiven zurückzuführen, die während der Sterilisation und/oder Aufbewahrung der Intraokularlinsen in Kontakt mit diesen treten können. Die Gleit-Additive sind im Allgemeinen in den verwendeten Materialien enthalten, die Gleit-Eigenschaften haben.

Ein gattungsgemäßes Intraokularlinsen-Aufbewahrungssystem ist aus der EP 2 286 764 A1 bekannt, die auch eine Intraokularlinsen-Übergabe-Anordnung und ein Verfahren zur Übergabe einer Intraokularlinse an eine Injektions-Einrichtung offenbart.

Die DE 10 2006 000 929 A1 offenbart außerdem ein bekanntes Intraokularlinsen-Aufbewahrungssystem.

Der Erfindung liegt daher die Aufgabe zugrunde, ein Intraokularlinsen-Aufbewahrungssystem zu schaffen, bei dem Biokompatibilitäts-Probleme im Wesentlichen ausschließbar sind. Ferner soll eine Intraokularlinsen-Übergabe-Anordnung bereitgestellt werden, bei der Biokompatibilitäts-Probleme im Wesentlichen ausschließbar sind. Es soll außerdem ein entsprechendes Intraokularlinsen-Übergabe-Verfahren geliefert werden, bei dem Biokompatibilitäts-Probleme im Wesentlichen ausschließbar sind. Diese Aufgabe wird erfindungsgemäß durch die in den unabhängigen Ansprüchen 1 und 13 angegebenen Merkmale gelöst. Der Kern der Erfindung liegt darin, dass die zu implantierende Intraokularlinse zunächst außerhalb der Injektions-Einrichtung angeordnet ist und beispielsweise bei geplanter Implantation unmittelbar aus dem Aufbewahrungs-Behältnis an die Injektions-Einrichtung durch die betätigbare Übergabe-Einrichtung übergeben bzw. überführt wird. Die Intraokularlinse ist unmittelbar aus dem Aufbewahrungs-Behältnis an die Injektions-Einrichtung durch die betätigbare Übergabe-Einrichtung übergebbar bzw. überführbar. Die Intraokularlinse ist in dem Aufbewahrungs-Behältnis sicher und steril aufbewahrbar.

Durch die Injektions-Einrichtungs-Haupt-Einführöffnung ist die Injektions-Einrichtung zumindest teilweise in das Innere des Aufbewahrungs-Behältnisses einführbar. Eine Injektions-Einrichtungs-Durchtritts-Öffnung, eine Injektions-Einrichtungs-Positionierungs-Einführöffnung und eine Injektions-Einrichtungs-Positionierungs-Ausführöffnung sind beabstandet zueinander um die Injektions-Einrichtungs-Haupt-Einführöffnung angeordnet und erstrecken sich jeweils seitlich bzw. radial von dieser weg. Es ist von Vorteil, wenn die Führungs-Flügel mit einer Injektions-Kartusche der Injektions-Einrichtung in fester Verbindung stehen.
Es ist von Vorteil, wenn das Intraokularlinsen-Aufbewahrungssystem aus mindestens einem thermoplastischen Kunststoff, wie Polypropylen, hergestellt ist.

Es ist zweckmäßig, wenn die Intraokularlinsen-Aufbewahrungs-Stellung und die Intraokularlinsen-Übergabe-Stellung jeweils End-Stellungen der Übergabe-Einrichtung sind. Die Intraokularlinsen-Aufbewahrungs-Stellung und die Intraokularlinsen-Übergabe-Stellung sind verschieden, das heißt zueinander räumlich beabstandet. Vorzugsweise ist eine stufenlose Bewegung der Übergabe-Einrichtung zwischen den Stellungen möglich. Es ist von Vorteil, wenn die Bewegung der Übergabe-Einrichtung geführt ist und eine entsprechende Lagerung vorgesehen ist.

Die Injektions-Einrichtung ist vorzugsweise als Injektor ausgebildet.

Es ist von Vorteil, wenn die Intraokularlinse ein optisches Teil und mindestens eine Haptik umfasst, die an dem optischen Teil angeordnet ist.

Die Intraokularlinse ist in der Intraokularlinsen-Aufnahme sicher, aber abgebbar, aufgenommen bzw. gehalten. Vorzugsweise ist die Intraokularlinse in der Intraokularlinsen-Aufnahme formschlüssig, bevorzugter durch Steckverbindung, Rastverbindung, Schnappverbindung oder dergleichen, gehalten.

Weitere vorteilhafte Ausgestaltungen der Erfindung sind in den Unteransprüchen angegeben.

Das mindestens eine Betätigungs-Mittel nach Anspruch 2 ist vorzugsweise als Druck-Knopf, Schiebe-Element, Zieh-Element, Freigabe-Element zur Freigabe einer gespeicherten Kraft, Schwenk-Element, Klinken-Element, elektrisches Schalt-Element oder dergleichen ausgebildet.

Die Ausgestaltung nach Anspruch 3 führt zu einer besonders einfach ausgebildeten und gut handhabbaren Übergabe-Einrichtung. Es ist von Vorteil, wenn das mindestens eine Betätigungs-Mittel als Angriffs-Element zum Wechselwirken mit der Injektions-Einrichtung bei deren Einführung in das Aufbewahrungs-Behältnis oder als Hebel-Arm ausgeführt ist. Vorzugsweise drückt ein Betätigungs-Vorsprung an der Injektions-Einrichtung auf das Betätigungs-Mittel, sodass die Übergabe-Einrichtung um eine Lager-Achse in ihre Intraokularlinsen-Übergabe-Stellung geschwenkt wird.

Gemäß Anspruch 4 ist die Übergabe-Einrichtung an dem Aufbewahrungs-Behältnis schwenkbar gelagert. Entsprechende Lager-Elemente sind dafür vorzugsweise an dem Aufbewahrungs-Behältnis vorgesehen bzw. angeordnet. Alternativ ist die Übergabe-Einrichtung an dem Aufbewahrungs-Behältnis verschiebbar gelagert.

Das mindestens eine Rückführungs-Feder-Mittel nach Anspruch 5 ist vorzugsweise als Feder-Element oder Feder-Materialblock ausgebildet. Es ist von Vorteil, wenn das Feder-Element ein Blatt-Feder-Element, eine Schrauben-Feder, eine Spiral-Feder, eine Torsions-Feder oder dergleichen ist. Es ist zweckmäßig, wenn das Blatt-Feder-Element vorgespannt ist oder sich bei einer Biegung spannt. Günstigerweise ist der Feder-Materialblock aus einem nachgiebigen Material gebildet, das nach Belastung wieder in seinen Ausgangs-Zustand automatisch zurückkehrt.

Es ist von Vorteil, wenn das Rückführungs-Feder-Mittel und die Übergabe-Einrichtung einteilig miteinander verbunden sind.

Der Injektions-Einrichtungs-Einführanschlag nach Anspruch 6 ist vorzugsweise an dem Aufbewahrungs-Behältnis oder der Übergabe-Einrichtung angeordnet. Vorzugsweise wirkt der Injektions-Einrichtungs-Einführanschlag mit einem entsprechenden Gegen-Anschlag an der Injektions-Einrichtung bei entsprechender Einführung der Injektions-Einrichtung in das Aufbewahrungs-Behältnis zusammen. Es ist von Vorteil, wenn der Injektions-Einrichtungs-Einführanschlag durch die Intraokularlinse selbst bzw. die Intraokularlinsen-Aufnahme gebildet ist und der Gegen-Anschlag durch eine Injektions-Kartusche der Injektions-Einrichtung gebildet ist.

Die mindestens eine Schwenkkulisse nach Anspruch 7 erzwingt eine Schwenk-Bewegung der zumindest teilweise eingeführten Injektions-Einrichtung um deren Längs-Achse für die Übergabe und Aufnahme der Intraokularlinse. Eine Übergabe der Intraokularlinse ist vorzugsweise nur dann möglich, wenn sich die Injektions-Einrichtung in ihrer verschwenkten Stellung befindet. Die mindestens eine Schwenkkulisse wirkt vorzugsweise mit einer entsprechenden Gegen-Fläche an der Injektions-Einrichtung beim Einführen zusammen.
Es ist von Vorteil, wenn das Aufbewahrungs-Behältnis einen Aufbewahrungs-Behältnis-Grundkörper und eine separate Verschluss-Einrichtung umfasst, wobei die Injektions-Einrichtungs-Haupt-Einführöffnung in der Verschluss-Einrichtung ausgebildet ist.
Das Aufbewahrungs-Behältnis weist also einen Aufbewahrungs-Behältnis-Grundkörper und eine Verschluss-Einrichtung auf, die separat ausgebildet ist und vorzugsweise auch wieder entfernbar von dem Aufbewahrungs-Behältnis-Grundkörper ist. Alternativ sind der Aufbewahrungs-Behältnis-Grundkörper und die Verschluss-Einrichtung einstückig und untrennbar miteinander ausgeführt.
Günstigerweise sind der Aufbewahrungs-Behältnis-Grundkörper und die Verschluss-Einrichtung fest miteinander verbunden, vorzugsweise miteinander verrastet.

Die Unteransprüche 2 bis 9 können auch Gegenstand des unabhängigen Anspruchs 13 sein.

Durch die Betätigung der Führungs-Flügel nach Anspruch 12 ist die Intraokularlinse von der Injektions-Kartusche greifbar und aufnehmbar, wobei sich vorzugsweise beim Verschwenken der Injektions-Einrichtung einer der Führungs-Flügel gegenüber dem anderen Führungs-Flügel verschwenkt und durch die weitere Öffnung in dem Aufbewahrungs-Behältnis wieder herausführbar ist. Nachfolgend wird unter Bezugnahme auf die beigefügte Zeichnung beispielhaft eine bevorzugte Ausführungsform der Erfindung beschrieben. Dabei zeigen:

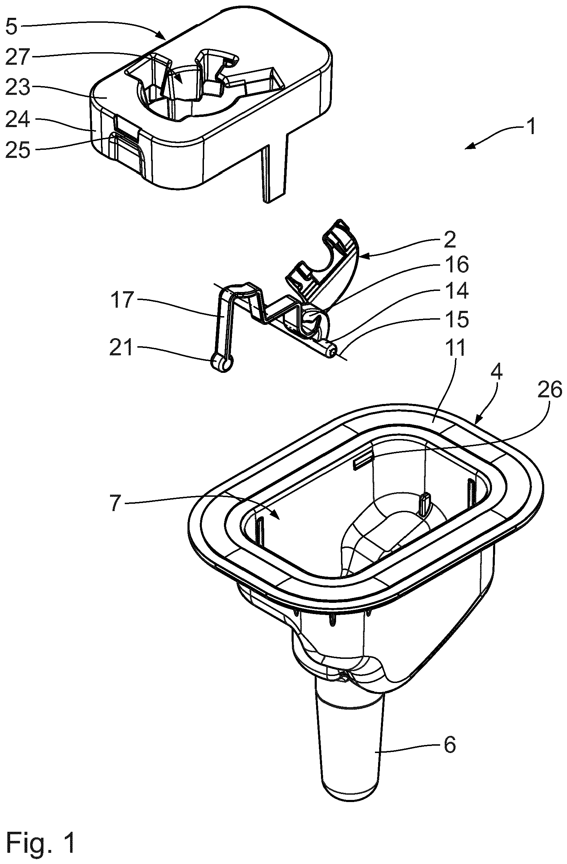

- Fig. 1: eine Explosions-Ansicht eines erfindungsgemäßen Intraokularlinsen-Aufbewahrungssystems,

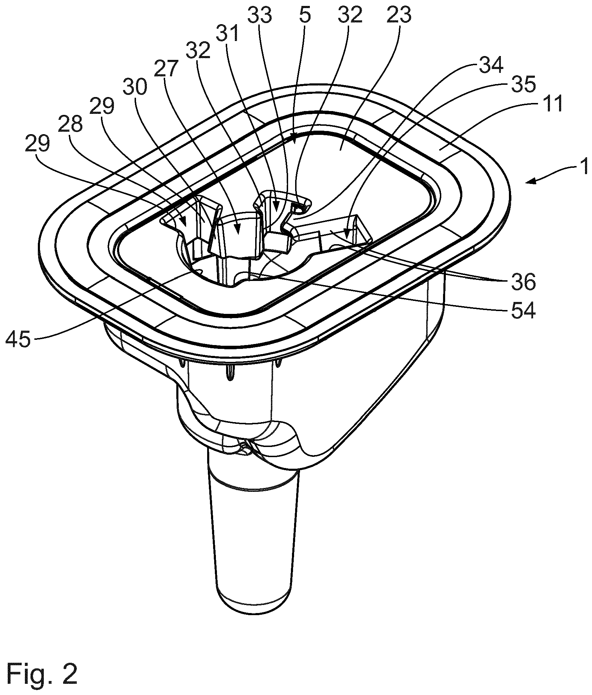

- Fig. 2: das in Fig. 1 dargestellte Intraokularlinsen-Aufbewahrungssystem im zusammengesetzten Zustand,

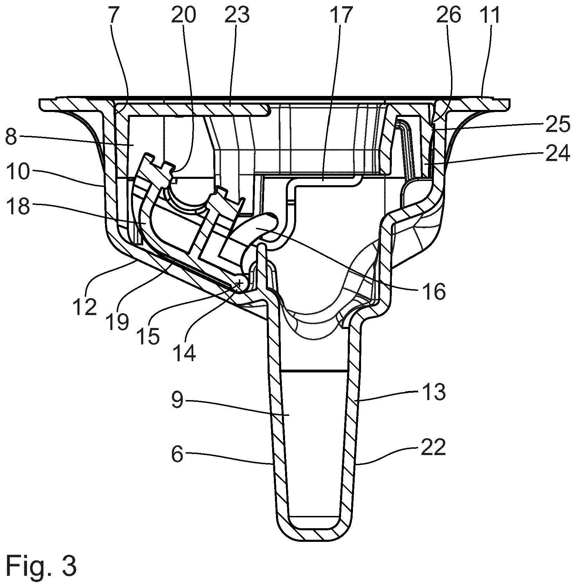

- Fig. 3: einen Längs-Schnitt durch das in Fig. 2 dargestellte Intraokularlinsen-Aufbewahrungssystem, wobei sich dessen Übergabe-Einrichtung in der Intraokularlinsen-Aufbewahrungs-Stellung befindet,

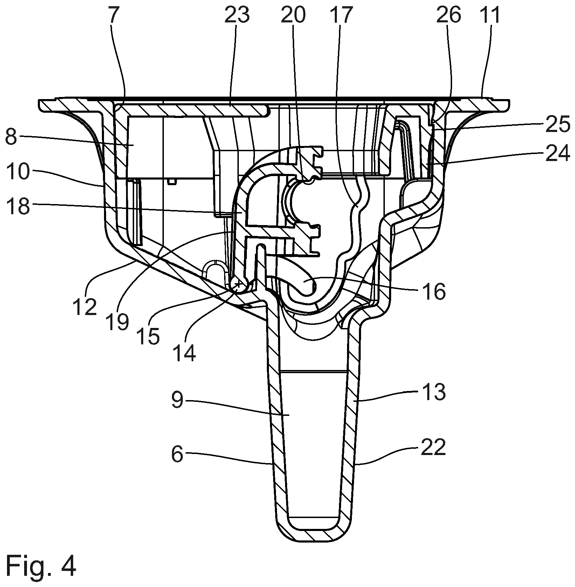

- Fig. 4: einen Längs-Schnitt durch das in Fig. 2 dargestellte Intraokularlinsen-Aufbewahrungssystem, wobei sich dessen Übergabe-Einrichtung in der Intraokularlinsen-Übergabe-Stellung befindet,
- Fig. 5: eine Draufsicht auf eine Intraokularlinsen-Übergabe-Anordnung mit dem in Fig. 2 dargestellten Intraokularlinsen-Aufbewahrungssystem, wobei sich die Injektions-Einrichtung in einer Ausgangs-Stellung befindet,
- Fig. 6: einen Längs-Schnitt durch die in Fig. 5 dargestellte Intraokularlinsen-Übergabe-Anordnung gemäß der Schnittlinie VI-VI in Fig. 5,

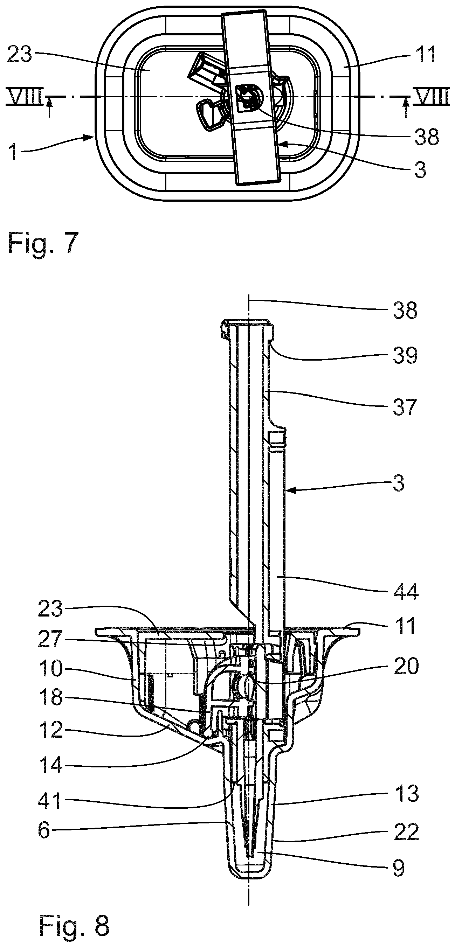

- Fig. 7: eine Draufsicht gemäß Fig. 5, wobei sich die Injektions-Einrichtung in einer Einführ-Stellung befindet,
- Fig. 8: einen Längs-Schnitt durch die in Fig. 7 dargestellte Intraokularlinsen-Übergabe-Anordnung gemäß der Schnittlinie VIII-VIII in Fig. 7,
- Fig. 9: eine Draufsicht gemäß Fig. 5, wobei sich die Injektions-Einrichtung in einer Übergabe-Stellung befindet,
- Fig. 10: einen Längs-Schnitt durch die in Fig. 9 dargestellte Intraokularlinsen-Übergabe-Anordnung gemäß der Schnittlinie X-X in Fig. 9,
- Fig. 11: eine Draufsicht gemäß Fig. 5, wobei sich die Injektions-Einrichtung in einer Auszieh-Stellung befindet, und
- Fig. 12: einen Längs-Schnitt durch die in Fig. 11 dargestellte Intraokularlinsen-Übergabe-Anordnung gemäß der Schnittlinie XII-XII in Fig. 11.

Ein Intraokularlinsen-Aufbewahrungssystem umfasst ein Aufbewahrungs-Behältnis 1 und eine in dem Aufbewahrungs-Behältnis 1 angeordnete Übergabe-Einrichtung 2, die zwischen einer Intraokularlinsen-Aufbewahrungs-Stellung und einer von der Intraokularlinsen-Aufbewahrungs-Stellung entfernten Intraokularlinsen-Übergabe-Stellung durch manuelle Betätigung beweglich ist. In der Intraokularlinsen-Aufbewahrungs-Stellung ist eine Intraokularlinse (nicht dargestellt) sicher und steril in dem Aufbewahrungs-Behältnis 1 aufbewahrt, während in der Intraokularlinsen-Übergabe-Stellung eine Übergabe der Intraokularlinse an eine Injektions-Einrichtung 3 möglich ist. Mittels der Injektions-Einrichtung 3 ist eine Implantation der Intraokularlinse in das Auge eines Patienten möglich.

Das Aufbewahrungs-Behältnis 1 umfasst einen Aufbewahrungs-Behältnis-Grundkörper 4 und eine Verschluss-Einrichtung 5, die im zusammengesetzten Zustand fest, aber vorzugsweise wieder lösbar, miteinander verbunden sind.

Der Aufbewahrungs-Behältnis-Grundkörper 4 hat eine äußere Wandung 6. Die Wandung 6 begrenzt eine Aufnahme-Öffnung 7, so dass dort der Aufbewahrungs-Behältnis-Grundkörper 4 nach außen offen ist. Der Aufbewahrungs-Behältnis-Grundkörper 4 hat einen ersten Teil-Abschnitt 8, der sich an die Aufnahme-Öffnung 7 anschließt und durch die Wandung 6 nach außen begrenzt ist. An den ersten Teil-Abschnitt 8 schließt sich wiederum ein zweiter Teil-Abschnitt 9 des Aufbewahrungs-Behältnis-Grundkörpers 4 an, der gegenüber dem ersten Teil-Abschnitt 8 verjüngt ist und durch die Wandung 6 nach außen begrenzt ist.

Im Bereich des ersten Teil-Abschnitts 8 ist der Aufbewahrungs-Behältnis-Grundkörper 4 nach seitlich außen durch eine Seiten-Wandung 10 begrenzt, die im Wesentlichen senkrecht zu einem endseitigen, flanschartigen Seiten-Steg 11 verläuft. Der Seiten-Steg 11 verläuft im Wesentlichen in einer Ebene. In dieser Ebene liegt auch die Aufnahme-Öffnung 7.

An die Seiten-Wandung 10 schließt sich ein Haupt-Boden 12 an, der gegenüberliegend zu der Aufnahme-Öffnung 7 angeordnet ist und vorzugsweise bereichsweise schräg zu der Seiten-Wandung 10 verläuft.

An den Haupt-Boden 12 schließt sich ein endseitig geschlossenes Vertiefungs-Stück 13 an, das im Wesentlichen rohrartig ausgeführt ist. Das Vertiefungs-Stück 13 erstreckt sich senkrecht zu der Ebene, in der die Aufnahme-Öffnung 7 liegt. Das Vertiefungs-Stück 13 hat eine QuerschnittsFläche, die kleiner, vorzugsweise wesentlich kleiner, als die Fläche der Aufnahme-Öffnung 7 ist. Das Vertiefungs-Stück 13 ist durch eine Vertiefungs-Steg-Wandung 22 begrenzt.

Die Übergabe-Einrichtung 2 ist einteilig. Sie hat ein Lager-Stück 14, das länglich ausgeführt ist und im Querschnitt im Wesentlichen kreisringartig bzw. kreisförmig ausgebildet ist. Mit dem Lager-Stück 14 ist umfangsseitig ein Kopplungs-Ansatz 16 fest verbunden, von welchem wiederum ein Rückführungs-Feder-Mittel 17 seitlich ausgeht. Das Rückführungs-Feder-Mittel 17 geht beabstandet zu dem Lager-Stück 14 von dem Kopplungs-Ansatz 16 aus und ist ursprünglich mehrfach abgewinkelt. Es ist länglich und leistenartig.

Ferner ist mit dem Lager-Stück 14 ein Intraokularlinsen-Aufnahme-Körper 18 umfangsseitig fest verbunden. Der Intraokularlinsen-Aufnahme-Körper 18 hat eine freie Anlage-Fläche bzw. -Kante 19, die vorzugsweise im Wesentlichen tangential zu dem Lager-Stück 14 verläuft. Gegenüberliegend zu der Anlage-Fläche 19 hat der Intraokularlinsen-Aufnahme-Körper 18 eine Intraokularlinsen-Aufnahme 20, die in senkrechter Richtung beabstandet zu der Anlage-Fläche 19 angeordnet ist. Wenn die Intraokularlinsen-Aufnahme 20 bestückt ist, ist in der Intraokularlinsen-Aufnahme 20 eine Intraokularlinse (nicht dargestellt) sicher, aber auch wieder abgebbar, gehalten.

Das Lager-Stück 14 ist in mindestens einer Lager-Aufnahme (nicht dargestellt) schwenkbar gehalten, die benachbart zu dem Vertiefungs-Stück 13 an dem Haupt-Boden 12 angebracht ist und eine Verschwenkung der Übergabe-Einrichtung 2 um eine Lager-Achse 15 erlaubt. Über die mindestens eine Lager-Aufnahme ist die Übergabe-Einrichtung 2 an dem Aufbewahrungs-Behältnis 1 bzw. dessen Haupt-Boden 12 gelagert. Die Übergabe-Einrichtung 2 ist wippenartig ausgeführt.
Das Rückführungs-Feder-Mittel 17 stützt sich dabei an dem Aufbewahrungs-Behältnis 1, genauer betrachtet in dem ersten Teil-Abschnitt 8, innenseitig ab. Es ist von Vorteil, wenn ein Bereich 21 des Rückführungs-Feder-Mittels 17 an dem Aufbewahrungs-Behältnis 1 räumlich festgelegt ist.
Die Wandung 6 ist durch die Seiten-Wandung 10, den Haupt-Boden 12 und die Vertiefungs-Steg-Wandung 22 gebildet.
In die Aufnahme-Öffnung 7 ist im montierten Zustand des Intraokularlinsen-Aufbewahrungssystems die Verschluss-Einrichtung 5 formschlüssig eingesetzt. Die Verschluss-Einrichtung 5 ist deckelartig und weist einen Deckel-Abschnitt 23 auf, der sich im eingesetzten Zustand der Verschluss-Einrichtung 5 in etwa in der Ebene des Seiten-Stegs 11 befindet und im Wesentlichen die Aufnahme-Öffnung 7 verschließt. Ferner hat die Verschluss-Einrichtung 5 einen Seiten-Steg 24, der ungefähr senkrecht zu dem Deckel-Abschnitt 23 verläuft und innenseitig mindestens bereichsweise an der Seiten-Wandung 10 anliegt. An dem Seiten-Steg 24 ist mindestens ein erstes Rast-Mittel 25 außenseitig angeordnet, das im eingesetzten Zustand der Verschluss-Einrichtung 5 mit einem entsprechenden Gegen-Rast-Mittel 26 an der Seiten-Wandung 10 rastend wechselwirkt.

In dem Deckel-Abschnitt 23 befindet sich eine Injektions-Einrichtungs-Haupt-Einführöffnung 27 die nach dem Vertiefungs-Stück 13 ausgerichtet ist und den Deckel-Abschnitt 23 vollständig durchdringt.

Von der Injektions-Einrichtungs-Haupt-Einführöffnung 27 geht seitlich eine Positionierungs-Einführöffnung 28 aus, die den Deckel-Abschnitt 23 vollständig durchdringt und sich von außen in Richtung auf den Haupt-Boden 12 des Aufbewahrungs-Behältnis-Grundkörpers 4 verjüngt. Die Positionierungs-Einführöffnung 28 ist so durch zwei einander gegenüberliegende Seiten-Wände 29 seitlich begrenzt, die an dem Deckel-Abschnitt 23 angeordnet sind und von außen in Richtung auf den Haupt-Boden 12 zusammenlaufen.

Zwischen einer der Seiten-Wände 29 und der Injektions-Einrichtungs-Haupt-Einführöffnung 27 ist ein Steg-Vorsprung 30 angeordnet, der in die Injektions-Einrichtungs-Haupt-Einführöffnung 27 seitlich ragt.

Ferner schließt sich an die Injektions-Einrichtungs-Haupt-Einführöffnung 27 seitlich eine Positionierungs-Ausführöffnung 31 an, die seitlich beabstandet zu der Positionierungs-Einführöffnung 28 in dem Deckel-Abschnitt 23 angeordnet ist und den Deckel-Abschnitt 23 vollständig durchdringt. Die Positionierungs-Ausführöffnung 31 ist wieder durch zwei einander gegenüberliegende Seiten-Wände 32 seitlich begrenzt. Die die Positionierungs-Ausführöffnung 31 begrenzenden Seiten-Wände 32 verlaufen im Wesentlichen parallel zueinander.

Zwischen der der Positionierungs-Einführöffnung 28 benachbarten Seiten-Wand 32 und der Injektions-Einrichtungs-Haupt-Einführöffnung 27 ist ein Steg-Vorsprung 33 im Bereich der Injektions-Einrichtungs-Haupt-Einführöffnung 27 an dem Deckel-Abschnitt 23 vorgesehen, der die Durchgangs-Verbindung zwischen der Positionierungs-Ausführöffnung 31 und der Injektions-Einrichtungs-Haupt-Einführöffnung 27 reduziert.

Ferner springt von dem Deckel-Abschnitt 23 ein Blockier-Vorsprung 34 seitlich von der der Positionierungs-Einführöffnung 28 entfernten Seiten-Wand 32 in Richtung auf die gegenüberliegende Seiten-Wand 32 vor. Der Blockier-Vorsprung 34 ist außenseitig bündig mit dem Deckel-Abschnitt 23, weist jedoch eine geringere Dicke als der Deckel-Abschnitt 23 auf.

Außerdem schließt sich eine Durchtritts-Öffnung 35 seitlich an die Injektions-Einrichtungs-Haupt-Einführöffnung 27 an. Die Durchtritts-Öffnung 35 ist in dem Deckel-Abschnitt 23 ausgebildet und durchdringt diesen vollständig. Die Positionierungs-Ausführöffnung 31 ist zwischen der Durchtritts-Öffnung 35 und der Positionierungs-Einführöffnung 28 angeordnet. Die Durchtritts-Öffnung 35 ist durch zwei einander gegenüberliegende Seiten-Wände 36 seitlich begrenzt, die parallel zueinander verlaufen.

An die Injektions-Einrichtungs-Haupt-Einführöffnung 27 schließt sich seitlich ferner eine Begrenzungs-Öffnung 45 an, die benachbart zu der Positionierungs-Einführöffnung 28 in dem Deckel-Abschnitt 23 angeordnet ist. Die Begrenzungs-Öffnung 45 ist zwischen der Positionierungs-Einführöffnung 28 und der Durchtritts-Öffnung 35 angeordnet.

Der Steg-Vorsprung 30 ist der Begrenzungs-Öffnung 45 zugewandt. Der Blockier-Vorsprung 34 springt im Wesentlichen von der Durchtritts-Öffnung 35 weg.

Wie bereits erwähnt, ist die in dem Intraokularlinsen-Aufbewahrungssystem aufbewahrte Intraokularlinse durch die Injektions-Einrichtung 3 in das Auge eines Patienten implantierbar. Die Injektions-Einrichtung 3 ist als Injektor ausgebildet und hat ein längliches, rohrartiges Gehäuse 37 sowie eine Längs-Mittel-Achse 38.

Die Injektions-Einrichtung 3 weist außerdem an einem ersten Ende 39 des Gehäuses 37 einen Halte- bzw. Abstütz-Vorsprung 40 auf, der gegenüber der Längs-Mittel-Achse 38 radial vorspringt. Ferner hat die Injektions-Einrichtung 3 ein zweites Ende 41. Benachbart zu dem zweiten Ende 41 ist in das Gehäuse 37 ein sich außenseitig verjüngender Verjüngungs-Körper 42 eingesetzt, der hohl ausgeführt ist.

Die Injektions-Einrichtung 3 hat außerdem eine Lade-Kammer 43, die zumindest teilweise seitlich offen ist und benachbart zu dem zweiten Ende 41 angeordnet ist. Ferner umfasst die Injektions-Einrichtung 3 eine Injektions-Kartusche 47, die in der Lade-Kammer 43 angeordnet ist.

Gegenüberliegend zu der Öffnung der Lade-Kammer 43 ist seitlich an dem Gehäuse 37 ein längs verlaufender Führungs-Steg 44 angeordnet.

In dem Gehäuse 37 ist ein Betätigungs-Kolben (nicht dargestellt) in Richtung auf das zweite Ende 41 verschiebbar geführt. Der Betätigungs-Kolben ist mit einer Kolben-Stange (nicht dargestellt) fest verbunden, die das Gehäuse 37 axial durchsetzt und gegenüber dem ersten Ende 39 nach außen vorsteht. Der Betätigungs-Kolben ist durch die Kolben-Stange betätigbar. Durch Verschiebung des Betätigungs-Kolbens ist eine in der Lade-Kammer 43 bzw. Injektions-Kartusche 47 angeordnete Intraokularlinse durch den Verjüngungs-Körper 42 führbar und dann in das Auge eines Patienten implantierbar.

Die Injektions-Kartusche 47 ist im Wesentlichen durch ein Rohrstück 49 gebildet, das sich in einer Einführ-Richtung der Injektions-Einrichtung 3 erstreckt. Das Rohrstück 49 begrenzt nach seitlich außen einen Intraokularlinsen-Aufnahmeraum 50. Es hat einen Mantel, der in der Längs-Richtung des Rohrstücks 49 aufgetrennt bzw. unterbrochen ist, sodass das Rohrstück 49 dort aufklappbar bzw. zusammenklappbar ist. Das Rohrstück 49 hat so durch die Längs-Unterbrechung zwei längs verlaufende Klappen-Bereiche mit freien End-Kanten 51 bzw. 52. Die End-Kanten 51, 52 sind im Wesentlichen benachbart zueinander angeordnet und liegen im Wesentlichen einander gegenüber. Das Rohrstück 49 ist im Querschnitt im Wesentlichen kreisbogenförmig. Das Rohrstück 49 ist als Klapp-Stück ausgeführt.

Die Injektions-Kartusche 47 hat außerdem einen ersten Führungs-Flügel 53 und einen zweiten Führungs-Flügel (nicht dargestellt), die im Wesentlichen plattenartig ausgeführt sind und sich nach seitlich außen bzw. in radialer Richtung zu der Längs-Mittel-Achse 38 erstrecken. An jedem Klappen-Bereich ist ein Führungs-Flügel 53 an dem Rohrstück 49 angeordnet. Die Anschluss-Bereiche der Führungs-Flügel 53 verlaufen parallel zu den End-Kanten 51, 52 und vorzugsweise auch benachbart zu diesen.

Das Intraokularlinsen-Aufbewahrungssystem und die Injektions-Einrichtung 3 bilden zusammen eine Intraokularlinsen-Übergabe-Anordnung.

Nachfolgend wird der Einsatz der Intraokularlinsen-Übergabe-Anordnung näher beschrieben.

Die Verschluss-Einrichtung 5 ist in die Aufnahme-Öffnung 7 eingesetzt und ist dort rastend an dem Aufbewahrungs-Behältnis-Grundkörper 4 festgelegt. Die Übergabe-Einrichtung 2 befindet sich in ihrer ursprünglichen Intraokularlinsen-Aufbewahrungs-Stellung. Dabei liegt die Anlage-Fläche 19 innenseitig an dem Haupt-Boden 12 an. In der Intraokularlinsen-Aufnahme 20 ist eine Intraokularlinse aufgenommen.

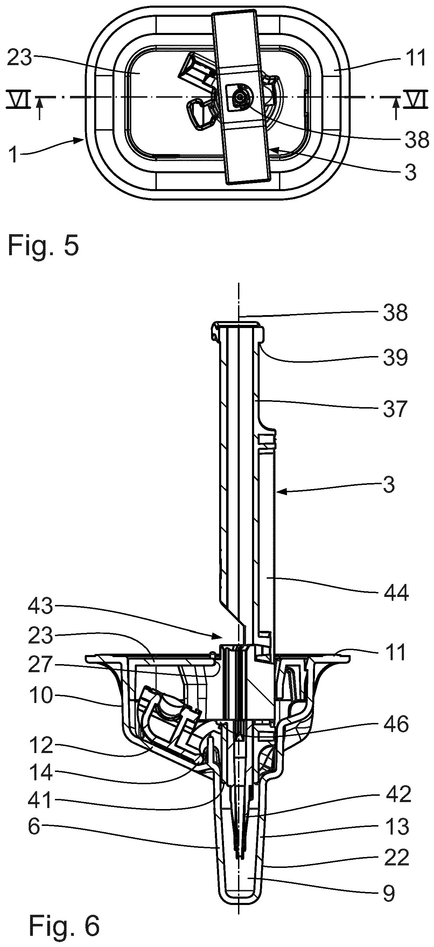

Gemäß Fig. 5, 6 ist die Injektions-Einrichtung 3 in das Aufbewahrungs-Behältnis 1 bereits teilweise eingeführt. Dabei durchdringt die Injektions-Einrichtung 3 mit ihrem Haupt-Körper die Injektions-Einrichtungs-Haupt-Einführöffnung 27 und ragt mit ihrem Verjüngungs-Körper 42 in das Vertiefungs-Stück 13. Die Injektions-Kartusche 47 befindet sich dabei in dem ersten Teil-Abschnitt 8 des Aufbewahrungs-Behältnisses 1. Die Injektions-Einrichtung 3 befindet sich in ihrer Ausgangs-Stellung. Die Intraokularlinsen-Übergabe-Anordnung kann so in diesem Zustand voreingestellt bzw. vormontiert bereitgestellt werden oder durch entsprechendes Einführen der Injektions-Einrichtung 3 geschaffen werden. Der Führungs-Steg 44 durchsetzt die Begrenzungs-Öffnung 45. Ein an der Injektions-Einrichtung 3 angeordneter und von dieser seitlich vorspringender Betätigungs-Vorsprung 46 liegt oben an dem Kopplungs-Ansatz 16 an.

Anschließend wird die Injektions-Einrichtung 3 weiter in das Aufbewahrungs-Behältnis 1 längs der Längs-Mittel-Achse 38 in einer Einführ-Richtung durch Aufbringen einer entsprechenden Einführ-Kraft eingeführt. Dabei drückt der Betätigungs-Vorsprung 46 auf den Kopplungs-Ansatz 16, so dass die Übergabe-Einrichtung 2 um die Lager-Achse 15 in ihre Intraokularlinsen-Übergabe-Stellung geschwenkt wird. Die Anlage-Fläche 19 wird von dem Haupt-Boden 12 abgehoben. Die Lager-Achse 15 verläuft senkrecht zu der Einführ-Richtung. Der Kopplungs-Ansatz 16 bildet also ein Betätigungs-Mittel zur Betätigung der Übergabe-Einrichtung 2. Dabei wird die Übergabe-Einrichtung 2 um etwa 15° bis 90°, bevorzugter um etwa 40° bis 75°, verschwenkt. In der Intraokularlinsen-Übergabe-Stellung befindet sich die Intraokularlinsen-Aufnahme 20 in der Injektions-Kartusche 47 bzw. benachbart zu dieser. Genauer betrachtet befindet sich die Intraokularlinse in der Intraokularlinsen-Übergabe-Stellung in der Übergabe-Einrichtung 2 unmittelbar vor dem Intraokularlinsen-Aufnahmeraum 50. Durch die Schwenk-Bewegung der Übergabe-Einrichtung 2 wird das längliche Rückführungs-Feder-Mittel 17 gebogen und gespannt. Es erzeugt so eine Rückführungs-Kraft. Ein weiteres axiales Einführen der Injektions-Einrichtung 3 wird durch einen Anschlag der Intraokularlinse an dem Rohrstück 49 verhindert. Der erste Führungs-Flügel 53 durchsetzt dabei die Durchtritts-Öffnung 35. Er ist dabei zwischen den beiden Seiten-Wänden 36 angeordnet, die ein Verschwenken der Injektions-Kartusche 47 um die Längs-Mittel-Achse 38 durch Anschlag an dem ersten Führungs-Flügel 53 verhindern.

Der zweite Führungs-Flügel durchsetzt dabei die Positionierungs-Einführöffnung 28. Die beiden Führungs-Flügel 53 sind für das Durchsetzen der Öffnungen 28, 35 entsprechend durch Aufbiegen des Rohrstücks 49 aufzuklappen bzw. auseinander zu schwenken, sodass eine Einführung der Führungs-Flügel 53 durch die Öffnungen 28, 35 möglich ist.

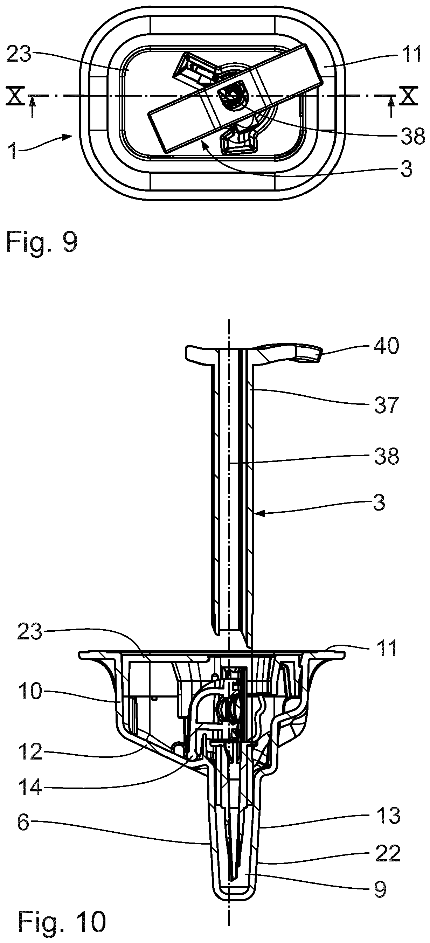

Bezugnehmend auf die Fig. 9 und 10 wird die Injektions-Einrichtung 3 anschließend um die Längs-Mittel-Achse 38 relativ zu dem Aufbewahrungs-Behältnis 1 manuell verschwenkt. Dabei dreht sich die Intraokularlinsen-Aufnahme 20 vollständig seitlich in die Injektions-Kartusche 47 der Injektions-Einrichtung 3 ein. Dabei wird die Intraokularlinse aus der Intraokularlinsen-Aufnahme 20 gezwängt, bevorzugter gedrückt, und an die Injektions-Einrichtung 3 bzw. deren Injektions-Kartusche 47 übergeben. Durch die Drehung der Injektions-Einrichtung 3 um die Längs-Mittel-Achse 38 werden die End-Kanten 51, 52 weiter aufeinander zu bewegt, sodass der Intraokularlinsen-Aufnahmeraum 50 weiter geschlossen wird. Dabei umgreifen die Klappen-Bereiche die Intraokularlinse. Diese Schwenk-Bewegung wird durch die Seiten-Wände 29 der Positionierungs-Einführöffnung 28 und die Seiten-Wände 32 der Positionierungs-Ausführöffnung 31 vorgegeben, die Schwenkkulissen bilden. Ein zu weites Schwenken in diese Richtung wird durch Kontakt des Führungs-Stegs 44 mit dem Steg-Vorsprung 30 verhindert. Ein gegensinniges Verschwenken wird durch den Kontakt einer Kontakt-Fläche 54 mit dem Steg-Vorsprung 30 verhindert. Die Kontakt-Fläche 54 begrenzt die Begrenzungs-Öffnung 45 seitlich. Sie ist an dem der Positionierungs-Einführöffnung 28 entfernten Ende der Begrenzungs-Öffnung 45 vorgesehen. Der Führungs-Steg 44 liegt dann an dem Steg-Vorsprung 30 an, so dass ein weiteres Verschwenken der Injektions-Einrichtung 3 gegenüber dem Aufbewahrungs-Behältnis 1 verhindert wird. Der Steg-Vorsprung 30 bildet so einen Schwenk-Anschlag.

Bei einer Rückbewegung der Übergabe-Einrichtung 2 in die Intraokularlinsen-Aufbewahrungs-Stellung wird die Intraokularlinse durch die Klappen-Bereiche aus der Übergabe-Einrichtung 2 gelöst und in den Intraokularlinsen-Aufnahmeraum 50 übergeben.

Nach Abschluss der Schwenk-Bewegung steht der durch die Positionierungs-Einführöffnung 28 eingeführte zweite Führungs-Flügel in einer zweiten, verschwenkten Position, sodass er nach der Positionierungs-Ausführöffnung 31 ausgerichtet ist.

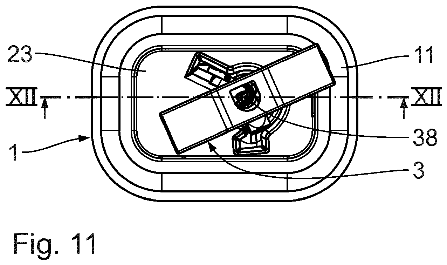

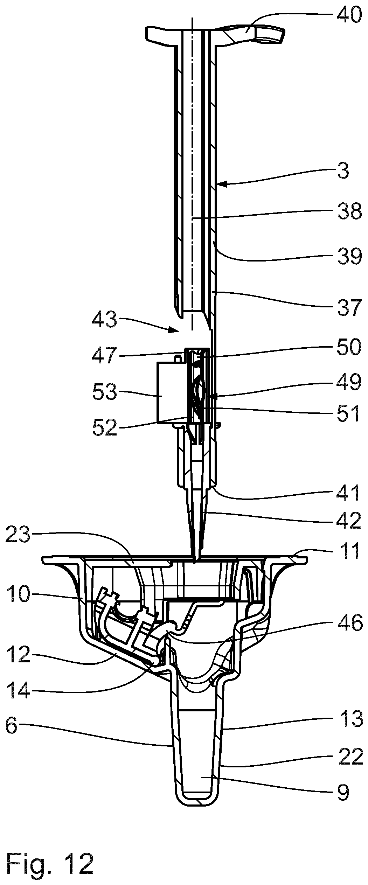

Anschließend wird die Injektions-Einrichtung 3 entgegen der Einführ-Richtung wieder aus dem Aufbewahrungs-Behältnis 1 entfernt (Fig. 11, 12). Die Intraokularlinse befindet sich nun in der Injektions-Einrichtung 3. Durch das Rückführungs-Feder-Mittel 17 wird die Übergabe-Einrichtung 2 wieder in ihre Intraokularlinsen-Aufbewahrungs-Stellung um die Lager-Achse 15 zurückgeschwenkt. Der zweite Führungs-Flügel durchsetzt dabei die Positionierungs-Ausführöffnung 31. Der erste Führungs-Flügel 53 durchsetzt wieder die Durchtritts-Öffnung 35.

Die Intraokularlinse kann nun mit der Injektions-Einrichtung 3 in das Auge eines Patienten implantiert werden.

## Patentansprüche

1. Intraokularlinsen-Aufbewahrungssystem zur Aufbewahrung einer Intraokularlinse, umfassend
a) ein Aufbewahrungs-Behältnis (1) zur Aufbewahrung der Intraokularlinse, und
b) eine in dem Aufbewahrungs-Behältnis (1) angeordnete, betätigbare Übergabe-Einrichtung (2) mit einer Intraokularlinsen-Aufnahme (20) zur Aufnahme der Intraokularlinse, wobei die Übergabe-Einrichtung (2) beweglich ist zwischen
i) einer Intraokularlinsen-Aufbewahrungs-Stellung zur Aufbewahrung der Intraokularlinse in dem Aufbewahrungs-Behältnis (1), und
ii) einer Intraokularlinsen-Übergabe-Stellung zur Übergabe der Intraokularlinse an eine Injektions-Einrichtung (3),
c) wobei in dem Aufbewahrungs-Behältnis (1) eine Injektions-Einrichtungs-Haupt-Einführöffnung (27) zur Einführung der Injektions-Einrichtung (3) in das Aufbewahrungs-Behältnis (1) ausgebildet ist,
**dadurch gekennzeichnet, dass**
d) sich seitlich an die Injektions-Einrichtungs-Haupt-Einführöffnung (27) anschließen
i) eine Injektions-Einrichtungs-Durchtritts-Öffnung (35) zum Ausrichten eines ersten Führungs-Flügels (53) der Injektions-Einrichtung (3) beim Einführen und
ii) eine Injektions-Einrichtungs-Positionierungs-Einführöffnung (28) zum Ausrichten eines zweiten Führungs-Flügels der Injektions-Einrichtung (3) beim Einführen sowie iii) eine Injektions-Einrichtungs-Positionierungs-Ausführöffnung (31) zum Ausrichten des zweiten Führungs-Flügels der Injektions-Einrichtung (3) beim Ausführen.

2. Intraokularlinsen-Aufbewahrungssystem nach Anspruch 1, **dadurch gekennzeichnet, dass** das Aufbewahrungs-Behältnis (1) mindestens ein mit der Übergabe-Einrichtung (2) gekoppeltes Betätigungs-Mittel zur Bewegung der Übergabe-Einrichtung (2) zwischen der Intraokularlinsen-Aufbewahrungs-Stellung und der Intraokularlinsen-Übergabe-Stellung aufweist

3. Intraokularlinsen-Aufbewahrungssystem nach Anspruch 1, **dadurch gekennzeichnet, dass** die Übergabe-Einrichtung (2) mindestens ein zu ihrer Bewegung zwischen der Intraokularlinsen-Aufbewahrungs-Stellung und der Intraokularlinsen-Übergabe-Stellung angeordnetes Betätigungs-Mittel (16) zur Betätigung durch Einführen der Injektions-Einrichtung (3) in das Aufbewahrungs-Behältnis (1) aufweist

4. Intraokularlinsen-Aufbewahrungssystem nach einem der vorherigen Ansprüche, **dadurch gekennzeichnet, dass** die Übergabe-Einrichtung (2) an dem Aufbewahrungs-Behältnis (1) schwenkbar gelagert ist.

5. Intraokularlinsen-Aufbewahrungssystem nach einem der vorherigen Ansprüche, **dadurch gekennzeichnet, dass** mit der Übergabe-Einrichtung (2) mindestens ein Rückführungs-Feder-Mittel (17) verbunden ist, das beim Bewegen der Übergabe-Einrichtung (2) in die Intraokularlinsen-Übergabe-Stellung eine Übergabe-Einrichtungs-Rückführungs-Kraft zur Rückführung der Übergabe-Einrichtung (2) in die Intraokularlinsen-Aufbewahrungs-Stellung erzeugt.

6. Intraokularlinsen-Aufbewahrungssystem nach einem der vorherigen Ansprüche, **gekennzeichnet durch** einen Injektions-Einrichtungs-Einführanschlag zur Begrenzung der Einführ-Bewegung der Injektions-Einrichtung (3) in das Aufbewahrungs-Behältnis (1).

7. Intraokularlinsen-Aufbewahrungssystem nach einem der vorherigen Ansprüche, **dadurch gekennzeichnet, dass** die die Injektions-Einrichtungs-Positionierungs-Einführöffnung (28) und die Injektions-Einrichtungs-Positionierungs-Ausführöffnung (31) begrenzenden Flächen (29, 32) mindestens eine Schwenkkulisse zum Erzwingen einer Schwenk-Bewegung der zumindest teilweise eingeführten Injektions-Einrichtung (3) um deren Längs-Mittel-Achse (38) relativ zu dem Aufbewahrungs-Behältnis (1) für die Übergabe der Intraokularlinse aufweisen.

8. Intraokularlinsen-Aufbewahrungssystem nach Anspruch 7, **dadurch gekennzeichnet, dass** die mindestens eine Schwenkkulisse an dem Aufbewahrungs-Behältnis (1) eine Schwenkkulissenfläche aufweist, die schräg und/oder quer zu einer Einführ-Richtung der Injektions-Einrichtung (3) in das Aufbewahrungs-Behältnis (1) verläuft.

9. Intraokularlinsen-Aufbewahrungssystem nach Anspruch 7 oder 8, **dadurch gekennzeichnet, dass** ein Schwenk-Anschlag (30) an dem Aufbewahrungs-Behältnis (1) die maximale Schwenk-Bewegung der Injektions-Einrichtung (3) relativ zu dem Aufbewahrungs-Behältnis (1) begrenzt.

10. Intraokularlinsen-Übergabe-Anordnung,
a) mit einem Intraokularlinsen-Aufbewahrungssystem nach einem der vorherigen Ansprüche, und
b) mit einer Injektions-Einrichtung (3) zur Einführung in das Aufbewahrungs-Behältnis (1) des Intraokularlinsen-Aufbewahrungssystems,
c) wobei die Übergabe-Einrichtung (2) in der Intraokularlinsen-Übergabe-Stellung die Intraokularlinse an die Injektions-Einrichtung (3) zur Implantation übergibt.

11. Intraokularlinsen-Übergabe-Anordnung nach Anspruch 10, **dadurch gekennzeichnet, dass** die Injektions-Einrichtung (3) eine Injektions-Kartusche (47) umfasst, wobei die Injektions-Kartusche (47) durch ein Klapp-Stück (49) mit zwei freien Klapp-Bereichen gebildet ist, die beweglich zueinander sind und jeweils einen nach seitlich außen verlaufenden Führungs-Flügel (53) tragen.

12. Intraokularlinsen-Übergabe-Anordnung nach Anspruch 11, **dadurch gekennzeichnet, dass** die Führungs-Flügel (53) beim Einführen der Injektions-Einrichtung (3) in dem Aufbewahrungs-Behältnis (1) die Injektions-Einrichtungs-Positionierungs-Einführöffnung (28) sowie die Injektions-Einrichtungs-Durchtritts-Öffnung (35) durchsetzen, die sich seitlich an die Injektions-Einrichtungs-Haupt-Einführöffnung (27) anschließen.

13. Verfahren zur Übergabe einer Intraokularlinse an eine Injektions-Einrichtung (3), umfassend
- Bereitstellen eines Intraokularlinsen-Aufbewahrungssystems nach einem der Ansprüche 1 bis 9, und
- Betätigen der Übergabe-Einrichtung (2) zur Bewegung derselben von der Intraokularlinsen-Aufbewahrungs-Stellung in die Intraokularlinsen-Übergabe-Stellung zur Übergabe der Intraokularlinse an die Injektions-Einrichtung (3).

## Claims

1. Intraocular lens storage system for storing an intraocular lens, comprising
a) a storage container (1) for storing the intraocular lens, and
b) an actuable transfer device (2), which is arranged in the storage container (1), with an intraocular lens receiver (20) for receiving the intraocular lens, wherein the transfer device (2) is movable between
i) an intraocular lens storage position for storing the intraocular lens in the storage container (1), and
ii) an intraocular lens transfer position for transferring the intraocular lens to an injection device (3)
c) wherein an injection device main introduction opening (27) for introducing the injection device (3) into the storage container (1) is formed in the storage container (1),
**characterized in that**
d) the injection device main introduction opening (27) is laterally adjoined by
i) an injection device through-opening (35) for aligning a first guide wing (53) of the injection device (3) during introduction, and
ii) an injection device positioning introduction opening (28) for aligning a second guide wing of the injection device (3) on introduction as well as
iii) an injection device positioning removal opening (31) to align the second guide wing of the injection device (3) on removal.

2. Intraocular lens storage system according to claim 1, **characterized in that** the storage container (1) has at least one actuating means coupled to the transfer device (2) for moving the transfer device (2) between the intraocular lens storage position and the intraocular lens transfer position.

3. Intraocular lens storage system according to claim 1, **characterized in that** the transfer device (2) has at least one actuating means (16), which is arranged for the movement thereof between the intraocular lens storage position and the intraocular lens transfer position, for actuation by introducing the injection device (3) into the storage container (1).

4. Intraocular lens storage system according to any one of the preceding claims, **characterized in that** the transfer device (2) is pivotably mounted on the storage container (1).

5. Intraocular lens storage system according to any one of the preceding claims, **characterized in that** at least one return spring means (17), which upon movement of the transfer device (2) into the intraocular lens transfer position produces a transfer device return force to return the transfer device (2) into the intraocular lens storage position, is connected to the transfer device (2).

6. Intraocular lens storage system according to any one of the preceding claims, **characterized by** an injection device introduction stop for limiting the introduction movement of the injection device (3) into the storage container (1).

7. Intraocular lens storage system according to any one of the preceding claims, **characterized in that** the faces (29, 32) limiting the injection device positioning introduction opening (28) and the injection device positioning removal opening (31) have at least one pivoting link for forcing a pivoting movement of the at least partially introduced injection device (3) about the longitudinal centre axis (38) thereof relative to the storage container (1) to transfer the intraocular lens.

8. Intraocular lens storage system according to claim 7, **characterized in that** the at least one pivoting link has a pivoting link face on the storage container (1), said pivoting link face running obliquely and/or transversely to an introduction direction of the injection device (3) into the storage container (1).

9. Intraocular lens storage system according to claim 7 or 8, **characterized in that** a pivot stop (30) on the storage container (1) limits the maximum pivoting movement of the injection device (3) relative to the storage container (1).

10. Intraocular lens transfer arrangement
a) with an intraocular lens storage system according to any one of the preceding claims, and
b) with an injection device (3) for introduction into the storage container (1) of the intraocular lens storage system,
c) wherein the transfer device (2) in the intraocular lens transfer position transfers the intraocular lens to the injection device (3) for implantation.

11. Intraocular lens transfer arrangement according to claim 10, **characterized in that** the injection device (3) comprises an injection cartridge (47), wherein the injection cartridge (47) is formed by a folding piece (49) with two free folding regions, which are movable in relation to one another and in each case carry a guide wing (53) running laterally outwardly.

12. Intraocular lens transfer arrangement according to claim 11, **characterized in that** the guide wings (53), on introduction of the injection device (3), pass through the injection device positioning introduction opening (28) and the injection device through-opening (35), said openings laterally adjoining the injection device main introduction opening (27).

13. Method for transferring an intraocular lens to an injection device (3), comprising
- providing an intraocular lens storage system according to any one of claims 1 to 9, and
- actuating the transfer device (2) to move it from the intraocular lens storage position into the intraocular lens transfer position to transfer the intraocular lens to the injection device (3).

## Revendications

1. Système de conservation de lentille intraoculaire permettant la conservation d'une lentille intraoculaire, comportant
a) un contenant de conservation (1) permettant la conservation de la lentille intraoculaire, et
b) un dispositif de transfert (2) pouvant être actionné et agencé dans le contenant de conservation (1) et pourvu d'un logement de lentille intraoculaire (20) permettant le logement de la lentille intraoculaire, dans lequel le dispositif de transfert (2) est mobile entre
i) une position de conservation de lentille intraoculaire permettant la conservation de la lentille intraoculaire dans le contenant de conservation (1), et
ii) une position de transfert de lentille intraoculaire permettant le transfert de la lentille intraoculaire sur un dispositif d'injection (3),
c) dans lequel un orifice d'introduction principal de dispositif d'injection (27) permettant l'introduction du dispositif d'injection (3) dans le contenant de conservation (1) est formé dans le contenant de conservation (1),
**caractérisé en ce que**
d) se situent latéralement dans le prolongement de l'orifice d'introduction principal de dispositif d'injection (27)
i) un orifice de passage de dispositif d'injection (35) permettant l'orientation d'une première ailette de guidage (53) du dispositif d'injection (3) lors de l'introduction et
ii) un orifice d'introduction pour le positionnement du dispositif d'injection (28) permettant l'orientation d'une deuxième ailette de guidage du dispositif d'injection (3) lors de l'introduction ainsi que
iii) un orifice d'évacuation pour le positionnement du dispositif d'injection (31) permettant l'orientation de la deuxième ailette de guidage du dispositif d'injection (3) lors de l'évacuation.

2. Système de conservation de lentille intraoculaire selon la revendication 1, **caractérisé en ce que** le contenant de conservation (1) comprend au moins un moyen d'actionnement accouplé au dispositif de transfert (2) et permettant le déplacement du dispositif de transfert (2) entre la position de conservation de lentille intraoculaire et la position de transfert de lentille intraoculaire.

3. Système de conservation de lentille intraoculaire selon la revendication 1, **caractérisé en ce que** le dispositif de transfert (2) comprend au moins un moyen d'actionnement (16) agencé pour le déplacement dudit dispositif entre la position de conservation de lentille intraoculaire et la position de transfert de lentille intraoculaire et permettant l'actionnement par introduction du dispositif d'injection (3) dans le contenant de conservation (1).

4. Système de conservation de lentille intraoculaire selon l'une quelconque des revendications précédentes, **caractérisé en ce que** le dispositif de transfert (2) est monté pivotant sur le contenant de conservation (1).

5. Système de conservation de lentille intraoculaire selon l'une quelconque des revendications précédentes, **caractérisé en ce qu'**au moins un moyen élastique de retour (17), qui, lors du déplacement du dispositif de transfert (2) dans la position de transfert de lentille intraoculaire, génère une force de retour de dispositif de transfert permettant le retour du dispositif de transfert (2) dans la position de conservation de lentille intraoculaire, est relié au dispositif de transfert (2).

6. Système de conservation de lentille intraoculaire selon l'une quelconque des revendications précédentes, **caractérisé par** une butée d'introduction de dispositif d'injection permettant la limitation du déplacement d'introduction du dispositif d'injection (3) dans le contenant de conservation (1).

7. Système de conservation de lentille intraoculaire selon l'une quelconque des revendications précédentes, **caractérisé en ce que** les surfaces (29, 32) délimitant l'orifice d'introduction pour le positionnement du dispositif d'injection (28) et l'orifice d'évacuation pour le positionnement du dispositif d'injection (31) comprennent au moins une coulisse pivotante permettant d'imposer un mouvement de pivotement au dispositif d'injection (3) introduit au moins en partie autour de l'axe longitudinal médian (38) de celui-ci par rapport au contenant de conservation (1) pour le transfert de la lentille intraoculaire.

8. Système de conservation de lentille intraoculaire selon la revendication 7, **caractérisé en ce que** la au moins une coulisse pivotante présente sur le contenant de conservation (1) une surface de coulisse pivotante qui s'étend de manière inclinée et/ou transversale par rapport à une direction d'introduction du dispositif d'injection (3) dans le contenant de conservation (1).

9. Système de conservation de lentille intraoculaire selon la revendication 7 ou 8, **caractérisé en ce qu'**une butée de pivotement (30) limite sur le contenant de conservation (1) le mouvement de pivotement maximal du dispositif d'injection (3) par rapport au contenant de conservation (1).

10. Ensemble de transfert de lentille intraoculaire,
a) comprenant un système de conservation de lentille intraoculaire selon l'une quelconque des revendications précédentes, et
b) comprenant un dispositif d'injection (3) permettant l'introduction dans le contenant de conservation (1) du système de conservation de lentille intraoculaire,
c) dans lequel le dispositif de transfert (2), dans la position de transfert de lentille intraoculaire, transfère la lentille intraoculaire sur le dispositif d'injection (3) aux fins d'implantation.

11. Ensemble de transfert de lentille intraoculaire selon la revendication 10, **caractérisé en ce que** le dispositif d'injection (3) comporte une cartouche d'injection (47), dans lequel la cartouche d'injection (47) est formée par une pièce rabattable (49) pourvue de deux zones de rabattement libres, qui sont mobiles l'une par rapport à l'autre et qui portent chacune une ailette de guidage (53) s'étendant latéralement vers l'extérieur.

12. Ensemble de transfert de lentille intraoculaire selon la revendication 11, **caractérisé en ce que** les ailettes de guidage (53), lors de l'introduction du dispositif d'injection (3) dans le contenant de conservation (1), traversent l'orifice d'introduction pour le positionnement du dispositif d'injection (28) ainsi que l'orifice de passage du dispositif d'injection (35), qui se situent latéralement dans le prolongement de l'orifice d'introduction principal du dispositif d'injection (27).

13. Procédé de transfert d'une lentille intraoculaire sur un dispositif d'injection (3), comportant
- la fourniture d'un système de conservation de lentille intraoculaire selon l'une des revendications 1 à 9, et
- l'actionnement du dispositif de transfert (2) pour déplacer celui-ci de la position de conservation de lentille intraoculaire dans la position de transfert de lentille intraoculaire pour transférer la lentille intraoculaire sur le dispositif d'injection (3).
